# EUROPEAN PATENT APPLICATION

(11) **EP 4 582 531 A1**
(43) Date of publication of application: **09.07.2025**
(21) Application number: 23859288.5
(22) Date of filing: 28.08.2023
(51) Int. Cl.: C12M 3/00, C12M 1/04, C12M 1/02, C12M 1/36

(54) **TEMPERATURE-ADJUSTABLE ANTI-CONDENSATION CULTURE APPARATUS AND FLOW CONTROL METHOD FOR WET CULTURE**

(30) Priority: 02.09.2022 CN 202211076823
(71) Applicant: HUA YUE MEDICAL TECHNOLOGY CO., LTD, Guangzhou, Guangdong 510535 (CN)
(72) Inventor: LI, Ting, Guangzhou, Guangdong 510535 (CN); LI, Hongfeng, Guangzhou, Guangdong 510535 (CN); SHI, Zhenzhi, Guangzhou, Guangdong 510535 (CN); TANG, Chaolong, Guangzhou, Guangdong 510535 (CN)
(74) Representative: Cicconetti, Andrea
(86) International application number: PCT/CN2023/115196
(87) International publication number: WO 2024/046257

(57) **Abstract**

A temperature-adjustable anti-condensation culture apparatus and a flow control method for wet culture. The apparatus comprises: an upper housing, a lower housing, a sealing metal groove, a culture turntable assembly, and a humidifying module; an air inlet is formed in an outer side surface of the lower housing and is connected to the humidifying module, and a shunting interface is arranged at the corresponding position of an inner surface of the lower housing and is connected to the sealing metal groove; the sealing metal groove surrounds an inner wall of the lower housing; the culture turntable assembly is located inside the lower housing and sequentially comprises, from top to bottom, a culture turntable for placing a plurality of culture dishes, a first heating sheet, and a temperature-equalizing plate; the culture turntable, the first heating sheet, and the temperature-equalizing plate are attached. The scheme of the present application can perform large-capacity and all-around wet culture in the culture apparatus under the condition that the uniformity of the gas concentration and humidity in the culture apparatus is guaranteed.

## Description

The present disclosure claims the priority to the Chinese patent application with the filling No. 202211076823.3 filed with the Chinese Patent Office on September 2, 2022, and entitled "TEMPERATURE-ADJUSTABLE ANTI-CONDENSATION CULTURE APPARATUS AND FLOW CONTROL METHOD FOR WET CULTURE", the contents of which are incorporated herein by reference in entirety.

### TECHNICAL FIELD

The present disclosure relates to the technical field of assisted reproductive equipment, and particularly to a temperature-adjustable anti-condensation culture apparatus and a flow control method for wet culture.

### BACKGROUND ART

At present, in the field of assisted reproduction, in vitro culture of embryos is performed using large box-type culture apparatuss or tabletop culture apparatuss, which provide a culture environment with the temperature, humidity, and gas concentration required for embryo culture and allow microscopic observation and imaging of embryos without damaging the culture environment. Existing time-lapse culture apparatuss on the market are classified into dry culture and wet culture according to whether the gas inside the culture apparatus is subjected to humidification treatment. This type of culture apparatus can provide a favorable temperature and gas environment for in vitro embryo culture. However, when an operator, such as a doctor, needs to perform microscopic observation of embryos, it is necessary to transfer the embryos from the culture apparatus to the microscope for observation to assess embryo development. During the microscopic observation operation, embryos are exposed to the external environment, causing a significant decrease in the temperature of the culture medium in which the embryos are located. The solubility of external gases differs greatly from that inside the culture apparatus, leading to changes in the pH value of the culture medium, thereby affecting embryo development. At present, most dry culture apparatuss control gas through intake pipelines without performing humidification treatment on the internal environment of the culture apparatus. However, wet culture apparatuss perform humidification treatment on the internal environment of the culture apparatus, typically by placing a water tray inside the culture apparatus, where the water inside the water tray evaporates automatically to increase the humidity of the internal environment of the culture apparatus.

Existing time-lapse culture apparatuss on the market generally have the problem of small capacity, as they can only humidify a single chamber independently, making it difficult to meet the requirements of large-capacity wet culture. The wet culture method generally increases the overall humidity inside the culture apparatus by enlarging the evaporation area of the water in the water tray or raising the internal temperature of the culture apparatus. However, these methods not only make it difficult to achieve more than 90% RH but also increase the risk of condensation forming inside the culture apparatus, which promotes bacterial growth.

### SUMMARY

The present disclosure provides a temperature-adjustable anti-condensation culture apparatus and a flow control method for wet culture, which enable large-capacity and comprehensive wet culture for the culture apparatus while ensuring the uniformity of gas concentration and humidity inside the culture apparatus.

A first aspect of the present disclosure provides a temperature-adjustable anti-condensation culture apparatus, including: an upper housing, a lower housing, a sealed metal groove, a culture turntable assembly, and a humidification module, wherein
a gas inlet is provided on an outer surface of a side edge of the lower housing and is connected to an external device;
the humidification module includes a humidification water bottle, a humidification drawer configured for placing the humidification water bottle, and a humidification box sleeved over the humidification drawer, wherein a sealing plug is provided on the humidification box and is connected to the gas inlet;
a flow distribution interface is provided on an inner surface of a side edge of the lower housing at a position corresponding to the gas inlet and is connected to the sealed metal groove, wherein the sealed metal groove is arranged to surround an inner wall of the lower housing;
multiple gas outlets are provided on a surface of the sealed metal groove, starting from the flow distribution interface, wherein sizes of the gas outlets gradually increase in sequence in a direction toward a hatch of the culture apparatus;
the culture turntable assembly is located inside the lower housing and includes, in sequence from top to bottom, a culture turntable for placing multiple culture dishes, a first heating sheet, and a temperature-equalizing plate, wherein the culture turntable, the first heating sheet, and the temperature-equalizing plate are attached to each other;
an inner surface of a bottom of the lower housing is in closely attaching with the temperature-equalizing plate, wherein an outer surface of the bottom of the lower housing is provided with a second heating sheet; and
an upper surface of the lower housing is rigidly connected to the upper housing.

In this way, by providing the sealing plug in the humidification module to connect with the gas inlet, the gas can pass through.

The gas subjected to humidification treatment in the humidification module is completely delivered into the culture apparatus, ensuring the sealing connection between the culture apparatus and the humidification module. The humidified gas is delivered into the culture apparatus through the flow distribution interface along the sealed metal groove. Since the sealed metal groove is arranged to surround the inner wall of the lower housing, the gas can be delivered to each culture dish on the culture turntable, ensuring humidification treatment for each culture dish on the culture turntable and meeting the requirements of large-capacity wet culture. Furthermore, the sizes of the gas outlets gradually increase in sequence in the direction toward the hatch of the culture apparatus, which prevents gas from completely flowing out at the flow distribution interface and ensures that the humidified gas reaches the hatch position of the culture apparatus, thereby improving the uniformity of gas concentration and humidity inside the culture apparatus. The first heating sheet is directly in attaching with the culture turntable. By controlling the temperature of the first heating sheet, the temperature of the culture turntable can be maintained at a constant value. The temperature-equalizing plate is directly attached to the first heating sheet, which can reduce the gap between the first heating sheet and the culture turntable and the temperature-equalizing plate, making the temperature more uniform and blocking the influence of the external environment temperature on the temperature of the first heating sheet. This provides heat insulation and energy storage functions, thus reducing the temperature differences between the culture dishes and improving the uniformity of temperature at different positions of the culture dishes placed on the turntable. The lower housing of the culture apparatus is in closely attaching with the temperature-equalizing plate, which provides a smaller gap with the culture turntable. A second heating sheet is provided on the outer surface of the bottom of the lower housing. By controlling the temperature of the second heating sheet, the spatial temperature of the culture apparatus can be maintained at a constant value. The temperature-adjustable anti-condensation culture apparatus provided in the embodiments of the present disclosure reduces the gap between the lower housing and the culture turntable assembly, thus lowering the temperature gradient difference between the culture turntable assembly and the lower housing of the culture apparatus, reducing heat dissipation of the culture turntable assembly, and making the temperature of the culture dishes more stable and uniform. The present disclosure provides a technical solution to enable large-capacity and comprehensive wet culture for the culture apparatus while ensuring the uniformity of gas concentration and humidity inside the culture apparatus.

In one implementation, a hatch structure including an L-shaped hatch and an L-shaped hatch cover panel is further provided at a front end of the culture apparatus, wherein
the front surface of the lower housing is rigidly connected to the L-shaped hatch, a longer-side panel of the L-shaped hatch is parallel to a bottom surface of the lower housing, and a shorter-side panel of the L-shaped hatch is directly in attaching with an outer surface of the lower housing;
the longer-side panel is provided with a perforation, wherein a size of the perforation is greater than a preset size range of the culture dish; and
the L-shaped hatch cover panel is in closely attaching with the L-shaped hatch, wherein an inner surface of the L-shaped hatch cover panel is provided with a third heating sheet.

In this way, by providing a perforation on the longer-side panel according to the size of the culture dish within a reserved range, the requirement for observing the cell culture state inside the culture dish can be met. Furthermore, by providing the third heating sheet on the inner surface of the hatch cover panel, the temperature uniformity inside the culture apparatus can be further ensured by controlling the temperature of the third heating sheet.

In one implementation, the gas outlets of the sealed metal groove are symmetrically distributed on the left and right sides relative to a centerline where the hatch of the culture apparatus is located.

In one implementation, a diameter size range of the gas outlets is 0.2 mm to 3 mm.

In one implementation, a snap is provided on a front surface of the humidification drawer, wherein
a water level observation window is provided below the snap, and
a semicircular opening is provided at a center of an upper portion on each of both sides of the humidification drawer.

In this way, by providing a snap on the front surface of the humidification drawer, the humidification drawer can be conveniently removed from the heater and the humidification box. By providing a water level observation window below the snap, the water level in the humidification water bottle can be monitored in real time, facilitating timely adjustment of the water amount. Furthermore, by providing semicircular openings at the upper portions on both sides of the humidification drawer, the humidification water bottle can be conveniently retrieved as needed, thus improving the overall operational convenience.

In one implementation, multiple fan-shaped culture dishes are provided on the culture turntable, wherein
the fan-shaped culture dishes are closely arranged in a circular pattern around a center of the culture turntable, each fan-shaped culture dish is provided with multiple culture microchambers for placing embryos, and the culture microchambers are arranged in a straight line.

In this way, by designing the culture dishes as fan-shaped and arranging each fan-shaped culture dish in a circular pattern around the center of the culture turntable, the space of the culture turntable can be maximized, thereby increasing the upper limit of the number of cells that can be cultured in a single cycle within the culture apparatus.

A second aspect of the present disclosure further provides a flow control method for wet culture, applicable to the temperature-adjustable anti-condensation culture apparatus as described above, including:
adding a target water amount to the humidification module according to a target humidity of the culture apparatus, wherein an input end of the humidification module is connected to a gas supply module, and an output end of the humidification module is connected to the gas inlet of the culture apparatus;
controlling the gas supply module to transmit gas to the humidification module at a first preset flow rate, so that the gas transmitted by the gas supply module is humidified in the humidification module and then reaches the interior of the culture apparatus via the gas outlets on the surface of the sealed metal groove, wherein the first preset flow rate has a flow rate range of 100 ml to 450 ml per minute; and
controlling, in response to detecting that an internal humidity of the culture apparatus has reached the target humidity, the gas supply module to transmit gas to the humidification module at a second preset flow rate, so that the internal humidity of the culture apparatus is maintained within a preset deviation range of the target humidity, wherein the second preset flow has a flow rate range of 50 ml to 230 ml per minute, and the target humidity is greater than or equal to 90% RH.

In this way, under the wet culture method, gas is first transmitted into the culture apparatus at a relatively high gas flow rate. The humidified gas is delivered to the entire interior of the culture apparatus along the sealed metal groove, and the size of the gas outlets gradually increases in the direction of the hatch of the culture apparatus. This arrangement can prevent all the gas from flowing out at the flow distribution interface. The gas after humidification treatment can reach every corner of the culture apparatus along the gas outlets, quickly exchanging gas with the internal gas of the culture apparatus. This ensures uniformity in both gas humidity and gas concentration inside the culture apparatus while rapidly achieving high saturation humidity. During the wet culture process, the uniformity of gas humidity inside the culture apparatus is less than or equal to 5% RH. When the internal humidity of the culture apparatus reaches the target humidity, the gas flow rate is adjusted. A lower gas flow rate is used to transmit gas into the culture apparatus, ensuring that the internal humidity of the culture apparatus remains within the preset deviation range of the target humidity at all times.

In one implementation, after the output end of the humidification module is connected to the gas inlet of the culture apparatus, the method further includes closing the hatch of the culture apparatus and verifying the sealing of the culture apparatus and the humidification module.

In one implementation, before controlling the gas supply module to transmit gas to the humidification module at the first preset flow rate, the method further includes real-time detection of the internal temperature of the culture apparatus and the temperature of the heater, wherein
the first heating sheet, the second heating sheet, the third heating sheet, and the heater are controlled to enter heating mode until a preset temperature is reached. The first heating sheet, the second heating sheet, the third heating sheet, and the heater remain at the preset temperature when the wet culture apparatus operates.

In a third aspect, the present disclosure further provides a terminal device including a memory, a processor, and a computer program stored on the memory and configured to be executed by the processor. The processor, when executing the computer program, implements the above-mentioned flow control method for wet culture.

In a fourth aspect, the present disclosure provides a computer-readable storage medium, wherein the computer-readable storage medium includes a stored computer program, and when the computer program is run, the device in which the computer-readable storage medium is located is controlled to execute the above-mentioned flow control method for wet culture.

### BRIEF DESCRIPTION OF DRAWINGS

FIG. 1 is an internal structural diagram of a temperature-adjustable anti-condensation culture apparatus provided in an embodiment of the present disclosure;
FIG. 2 is a front structural diagram of a temperature-adjustable anti-condensation culture apparatus provided in an embodiment of the present disclosure;
FIG. 3 is an internal structural diagram of a humidification module provided in an embodiment of the present disclosure;
FIG. 4 is an internal structural diagram of a culture dish provided in an embodiment of the present disclosure; and
FIG. 5 is a flowchart of a flow control method for wet culture provided in an embodiment of the present disclosure.

### DETAILED DESCRIPTION OF EMBODIMENTS

The specific implementation of the present disclosure is described in further detail below in connection with the drawings and embodiments. The following examples are used to illustrate the present disclosure but are not used to limit the scope of the present disclosure.

The terms "first", "second", and so on in the specification and claims of the present disclosure or in the drawings are used to distinguish between different objects and are not intended to describe a specific sequence. In addition, the terms "include", "comprise", and any variations thereof, are intended to cover a non-exclusive inclusion. For example, a process, method, system, product, or apparatus comprising a series of steps or units is not limited to the listed steps or units, but may optionally include unlisted steps or units, or may optionally include other steps or units inherent to the process, method, product or apparatus.

The term "embodiment" used herein means that specific features, structures, or characteristics described in conjunction with the embodiment can be included in at least one embodiment of the present disclosure. The use of the phrase in various parts of the summary does not necessarily refer to the same embodiment, nor does it imply that these embodiments are mutually exclusive or independent of each other. Those skilled in the art understand explicitly and implicitly that the embodiments described herein can be combined with other embodiments.

### Embodiment 1

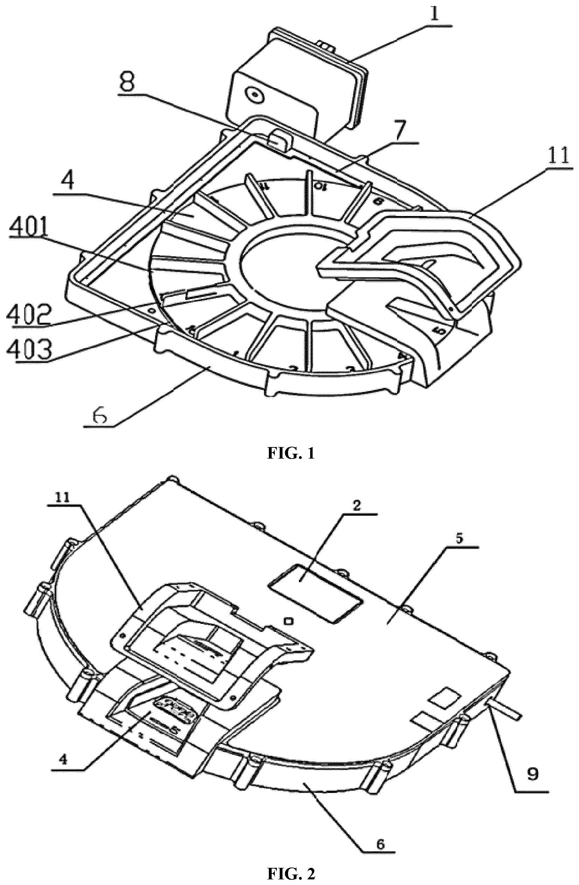

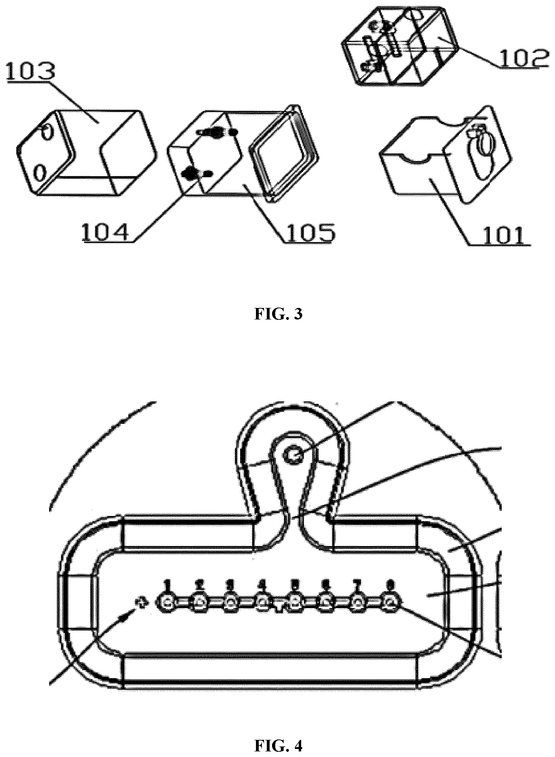

Referring to FIGS. 1, 2, and 3, FIG. 1 is an internal structural diagram of a temperature-adjustable anti-condensation culture apparatus provided in an embodiment of the present disclosure; FIG. 2 is a front structural diagram of a temperature-adjustable anti-condensation culture apparatus provided in an embodiment of the present disclosure; and FIG. 3 is an internal structural diagram of a humidification module provided in an embodiment of the present disclosure. The embodiment of the present disclosure provides a temperature-adjustable anti-condensation culture apparatus, including: an upper housing 5, a lower housing 6, a sealed metal groove 7, a culture turntable assembly 4, and a humidification module 1, wherein
a gas inlet 9 is provided on an outer surface of a side edge of the lower housing 6 and is connected to an external device;
the humidification module 1 includes a humidification water bottle 102, a humidification drawer 101 configured for placing the humidification water bottle, and a humidification box 105 sleeved over the humidification drawer 101, wherein a sealing plug 104 is provided on the humidification box 105 and is connected to the gas inlet 9;
a flow distribution interface 8 is provided on an inner surface of a side edge of the lower housing 6 at a position corresponding to the gas inlet and is connected to the sealed metal groove 7, wherein the sealed metal groove is arranged to surround an inner wall of the lower housing 6;
multiple gas outlets are provided on a surface of the sealed metal groove 7, starting from the flow distribution interface 8, wherein sizes of the gas outlets gradually increase in sequence in a direction toward a hatch of the culture apparatus;
the culture turntable assembly 4 is located inside the lower housing 6 and includes, in sequence from top to bottom, a culture turntable 401 for placing multiple culture dishes, a first heating sheet 402, and a temperature-equalizing plate 403, wherein the culture turntable 401, the first heating sheet 402, and the temperature-equalizing plate 403 are in directly attaching;
an inner surface of a bottom of the lower housing 6 is in closely attaching with the temperature-equalizing plate 403, wherein an outer surface of the bottom of the lower housing is provided with a second heating sheet; and
an upper surface of the lower housing 6 is rigidly connected to the upper housing 5.

By arranging a sealing plug in the humidification module to connect to the gas inlet, the gas subjected to humidification treatment in the humidification module can be ensured to be completely delivered into the culture apparatus, thus ensuring the sealing connection between the culture apparatus and the humidification module. The humidified gas is delivered into the culture apparatus through the flow distribution interface along the sealed metal groove. Since the sealed metal groove is arranged to surround the inner wall of the lower housing, the gas can be delivered to each culture dish on the culture turntable. Furthermore, the sizes of the gas outlets gradually increase in sequence in the direction toward the hatch of the culture apparatus, which prevents gas from completely flowing out at the flow distribution interface and ensures that the humidified gas reaches the hatch position of the culture apparatus, thereby improving the uniformity of gas concentration and humidity inside the culture apparatus. The first heating sheet is directly in attaching with the culture turntable. By controlling the temperature of the first heating sheet, the temperature of the culture turntable can be maintained at a constant value. The temperature-equalizing plate is directly attached to the first heating sheet, which can reduce the gap between the first heating sheet and the culture turntable and the temperature-equalizing plate, making the temperature more uniform and blocking the influence of the external environment temperature on the temperature of the first heating sheet. This provides heat insulation and energy storage functions, thus reducing the temperature differences between the culture dishes and improving the uniformity of temperature at different positions of the culture dishes placed on the turntable. The lower housing of the culture apparatus is in closely attaching with the temperature-equalizing plate, which provides a smaller gap with the culture turntable. A second heating sheet is provided on the outer surface of the bottom of the lower housing. By controlling the temperature of the second heating sheet, the spatial temperature of the culture apparatus can be maintained at a constant value. This lowers the temperature gradient difference between the culture turntable and the lower housing, reduces heat dissipation of the culture turntable, and makes the temperature of the turntable more stable and uniform.

In one embodiment, a hatch structure 11 including an L-shaped hatch and an L-shaped hatch cover panel is further provided at a front end of the culture apparatus, wherein the front surface of the lower housing is rigidly connected to the L-shaped hatch, a longer-side panel of the L-shaped hatch is parallel to a bottom surface of the lower housing, and a shorter-side panel of the L-shaped hatch is directly in attaching with an outer surface of the lower housing; the longer-side panel is provided with a perforation, wherein a size of the perforation is greater than a preset size range of the culture dish; and the L-shaped hatch cover panel is in closely attaching with the L-shaped hatch, wherein an inner surface of the L-shaped hatch cover panel is provided with a third heating sheet.

In this way, by providing a perforation on the longer-side panel according to the size of the culture dish within a reserved range, the requirement for observing the cell culture state inside the culture dish can be met. Furthermore, by providing the third heating sheet on the inner surface of the hatch cover panel, the temperature uniformity inside the culture apparatus can be further ensured by controlling the temperature of the third heating sheet.

In one embodiment, the gas outlets of the sealed metal groove 7 are symmetrically and evenly distributed on the left and right sides relative to a centerline where the hatch of the culture apparatus is located. Further, the diameter size range of the gas outlets is (0.2-3) mm. The quantity and aperture size of the gas outlets can be arranged according to the target humidity effect.

Preferably, taking the arrangement of 15 gas outlets on the sealed metal groove as an example, the aperture size of the gas outlets can be arranged within the range of (0.2-3) mm according to a ratio of 1:2:3. As a gas outlet arrangement solution of the present disclosure, five apertures with a diameter of 0.2 mm are uniformly arranged on the straight edge connected to the gas outlets (i.e., the straight edge opposite to the hatch direction inside the culture apparatus), two apertures with a diameter of 0.4 mm are uniformly arranged on each of the right-angled edges on the left and right sides of the hatch, and three apertures with a diameter of 0.6 mm are uniformly arranged on each of the arcuate edges on the left and right sides near the front hatch of the culture apparatus. As another gas outlet arrangement solution of the embodiment of the present disclosure, five apertures with a diameter of 0.5 mm are uniformly arranged on the straight edge connected to the gas outlets (i.e., the straight edge opposite to the hatch direction inside the culture apparatus), two apertures with a diameter of 1mm are uniformly arranged on each of the right-angled edges on the left and right sides, and three apertures with a diameter of 1.5 mm are uniformly arranged on each of the arcuate edges on the left and right sides near the front hatch of the culture apparatus. As a gas outlet arrangement solution of the embodiment of the present disclosure, five apertures with a diameter of 1mm are uniformly arranged on the straight edge connected to the gas outlets (i.e., the straight edge opposite to the hatch direction inside the culture apparatus), two apertures with a diameter of 2 mm are uniformly arranged on each of the right-angled edges on the left and right sides, and three apertures with a diameter of 3 mm are arranged on each of the arcuate edges on the left and right sides near the front hatch of the culture apparatus. It should be noted that the above gas outlet arrangement solutions are merely exemplary embodiments for reference and are not intended to limit the position and size arrangement of the gas outlets. It is only necessary to ensure that the gas inside the culture apparatus can be fully mixed so that the humidity inside the culture apparatus rapidly reaches the target humidity requirement. The specific quantity and aperture size combination of the gas outlets can be adaptively adjusted according to the target humidity requirement.

In one embodiment, the upper housing 5 and the lower housing 6 are hollowed out at a preset position and embedded with a transparent heating glass 2. Preferably, when microscopic observation of embryos inside the culture dish is required, the region of the transparent heating glass can serve as an observation window, thus reducing unnecessary hatch openings, lowering the risk of bacterial growth, and improving the overall safety of the culture apparatus. When gas enters the interior of the culture apparatus, heating of the glass can be controlled to prevent the gas from liquefying upon contact with the cold glass surface, thereby avoiding condensation and further ensuring the uniformity of gas humidity and temperature inside the culture apparatus.

In one embodiment, the culture turntable 401 passes through the center of the lower housing 6 via a connecting assembly and is connected to a rotation motor. Preferably, the culture turntable passes through the center of the lower housing via screws and is connected to the rotation motor module. By driving the rotating motor, the culture turntable can rotate, thereby allowing the observation window of the heating glass to capture the condition of all culture dishes on the culture turntable. In one embodiment, the humidification module 1 includes a humidification drawer 101, a humidification water bottle 102, a heater 103, a sealing plug 104, and a humidification box 105. Specifically, the humidification drawer 101 is configured to place the humidification water bottle 102. The heater 103 and the humidification box 105 are sequentially sleeved over the humidification drawer 101, and the bottom of the humidification box 105 is provided with two sealing plugs for connection with the gas inlet 9. The heater 103 is nested on the inner surface of the humidification box 105 and is in closely attaching with the humidification drawer 101. A layer of heating sheet is uniformly attached to the inner wall of the humidification box. By heating through the heating sheet, heat is transferred to the humidification water bottle, allowing the liquid inside the humidification water bottle to be heated and maintained at a stable temperature, generating water vapor. At this time, the heat generated by the heating sheet is greater than the heat carried away by the gas, keeping the gas in a heated state, thereby further ensuring that the gas entering the interior of the culture apparatus does not produce condensation. The humidification box, the heater, and the humidification drawer are sequentially nested, which allows the heat generated by the heater to be uniformly transferred to the humidification water bottle, thereby providing heat preservation and uniform heat transfer.

In one embodiment, the body of the humidification water bottle 102 is provided with a water level line. The water level line allows for the adjustment of the water volume, thus enabling precise control of the water injection amount.

In one embodiment, a snap is provided on a front surface of the humidification drawer 101, wherein a water level observation window is provided below the snap, and a semicircular opening is provided at a center of an upper portion on each of both sides of the humidification drawer 101. By providing a snap on the front surface of the humidification drawer, the humidification drawer can be conveniently removed from the heater and the humidification box. By providing a water level observation window below the snap, the water level in the humidification water bottle can be monitored in real time, facilitating timely adjustment of the water amount. Furthermore, by providing semicircular openings at the upper portions on both sides of the humidification drawer, the humidification water bottle can be conveniently retrieved as needed, thus improving the overall operational convenience.

Referring to FIG. 4, FIG. 4 is an internal structural diagram of a culture dish provided in an embodiment of the present disclosure. In one embodiment, multiple fan-shaped culture dishes are provided on the culture turntable 4, wherein the fan-shaped culture dishes are closely arranged in a circular pattern around a center of the culture turntable, each fan-shaped culture dish is provided with multiple culture microchambers for placing embryos, and the culture microchambers are arranged in a straight line. In the prior art, the culture turntable in the culture apparatus can generally arrange 6-8 culture dishes. However, in the embodiment of the present disclosure, by designing the culture dishes as fan-shaped and arranging each fan-shaped culture dish in a circular pattern around the center of the culture turntable, the space of the culture turntable can be maximized, thereby increasing the upper limit of the number of cells that can be cultured in a single cycle within the culture apparatus. Furthermore, a target number of culture microchambers are arranged in the culture dish according to culture requirements. A culture dish provided by an embodiment of the present disclosure can allow 8-16 embryos to be cultured at a single time.

The embodiment of the present disclosure provides a temperature-adjustable culture apparatus. By arranging a sealing plug in the humidification module to connect to the gas inlet, the gas subjected to humidification treatment in the humidification module can be ensured to be completely delivered into the culture apparatus, thus ensuring the sealing connection between the culture apparatus and the humidification module. The humidified gas is delivered into the culture apparatus through the flow distribution interface along the sealed metal groove. Since the sealed metal groove is arranged to surround the inner wall of the lower housing, the gas can be delivered to each culture dish on the culture turntable, ensuring humidification treatment for each culture dish on the culture turntable and meeting the requirements of large-capacity wet culture. Furthermore, the sizes of the gas outlets gradually increase in sequence in the direction toward the hatch of the culture apparatus, which prevents gas from completely flowing out at the flow distribution interface and ensures that the humidified gas reaches the hatch position of the culture apparatus, thereby improving the uniformity of gas concentration and humidity inside the culture apparatus. The first heating sheet is directly in attaching with the culture turntable. By controlling the temperature of the first heating sheet, the temperature of the culture turntable can be maintained at a constant value. The temperature-equalizing plate is directly attached to the first heating sheet, which can reduce the gap between the first heating sheet and the culture turntable and the temperature-equalizing plate, making the temperature more uniform and blocking the influence of the external environment temperature on the temperature of the first heating sheet. This provides heat insulation and energy storage functions, thus reducing the temperature differences between the culture dishes and improving the uniformity of temperature at different positions of the culture dishes placed on the turntable. The lower housing of the culture apparatus is in closely attaching with the temperature-equalizing plate, which provides a smaller gap with the culture turntable. A second heating sheet is provided on the outer surface of the bottom of the lower housing. By controlling the temperature of the second heating sheet, the spatial temperature of the culture apparatus can be maintained at a constant value. This lowers the temperature gradient difference between the culture turntable and the lower housing, reduces heat dissipation of the culture turntable, and makes the temperature of the turntable more stable and uniform. The temperature-adjustable anti-condensation culture apparatus provided in the embodiments of the present disclosure enables large-capacity and comprehensive wet culture for the culture apparatus while ensuring the uniformity of gas concentration and humidity inside the culture apparatus.

### Embodiment 2

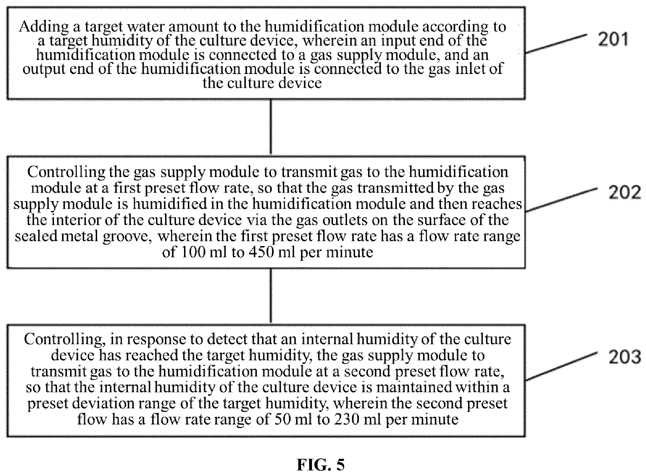

Referring to FIG.5, FIG. 5 is a flowchart of a flow control method for wet culture provided in an embodiment of the present disclosure. The embodiments of the present disclosure further provide a flow control method for wet culture, which includes steps 201 to 203. The specific steps are as follows.

Step 201: adding a target water amount to the humidification module according to a target humidity of the culture apparatus, wherein an input end of the humidification module is connected to a gas supply module, and an output end of the humidification module is connected to the gas inlet of the culture apparatus.

When the culture method is wet culture, the corresponding external gas supply pipeline is connected according to the gas supply mode of the culture apparatus to provide an external gas source for the culture apparatus. The target humidity of the culture apparatus is determined, and the corresponding target water volume is injected into the humidification water bottle. A connecting assembly is used to connect the output end of the gas supply module to the humidification module, and the sealing plug of the humidification module is tightly connected to the gas inlet of the culture apparatus.

In one embodiment, after the output end of the humidification module is connected to the gas inlet of the culture apparatus, the method further includes closing the hatch of the culture apparatus and verifying the sealing of the culture apparatus. After the connection of each component is completed, the hatch of the culture apparatus is closed, and the sealing performance of the culture apparatus is checked to ensure the gas transmission effect after being processed by the humidification module.

As a preferred solution of the embodiments of the present disclosure, when the culture method is dry culture, the corresponding external gas supply pipeline can be selected according to the gas supply mode and the external gas supply pipeline is connected to the gas inlet of the culture apparatus. The external gas supply pipeline directly provides a gas source for the culture apparatus to perform dry culture.

Step 202: controlling the gas supply module to transmit gas to the humidification module at a first preset flow rate, so that the gas transmitted by the gas supply module is humidified in the humidification module and then reaches the interior of the culture apparatus via the gas outlets on the surface of the sealed metal groove, wherein the first preset flow rate has a flow rate range of 100 ml to 450 ml per minute.

In one embodiment, before controlling the gas supply module to transmit gas to the humidification module at the first preset flow rate, the method further includes real-time detection of the internal temperature of the culture apparatus and the temperature of the heater, wherein the first heating sheet, the second heating sheet, the third heating sheet, and the heater are controlled to enter heating mode until a preset temperature is reached. The first heating sheet, the second heating sheet, the third heating sheet, and the heater remain at the preset temperature when the wet culture apparatus operates. After confirming the good sealing performance of the culture apparatus, in the embodiments of the present disclosure, the first heating sheet, the second heating sheet, the third heating sheet, and the heater are controlled to activate the heating mode, heating to 37 °C ± 0.1 °C. This stabilizes the internal temperature of the humidification module and the culture apparatus at 37°C ± 0.1 °C, thus preventing condensation caused by temperature differences when the device is filled with gas and humidity rises. Even under a high saturation humidity of 94%RH, no condensation occurs, ensuring that the temperature and humidity of the gas remain uniform and stable.

In one embodiment, the target value difference of the temperature between the lower housing and the culture turntable is controlled to be ≤1 °C, which ensures that the internal space temperature of the culture apparatus remains within the target value of ±0.1 °C, thereby maintaining a constant temperature inside the device.

Step 203: controlling, in response to detecting that an internal humidity of the culture apparatus has reached the target humidity, the gas supply module to transmit gas to the humidification module at a second preset flow rate, so that the internal humidity of the culture apparatus is maintained within a preset deviation range of the target humidity, wherein the second preset flow has a flow rate range of 50 ml to 230 ml per minute, and the target humidity is greater than or equal to 90 % RH.

As a preferred solution of the embodiment of the present disclosure, the second preset flow rate is preferably 1/2 or 1/3 of the first preset flow rate, particularly 1/2 of the first preset flow rate. Preferably, when the internal humidity of the culture apparatus is rapidly increased to 85%RH using a high-flow first preset flow rate of (100-150) ml/min, the second preset flow rate of (50-80) ml/min is adopted to transport gas into the culture apparatus, thereby maintaining the internal humidity of the culture apparatus within the range of (75-85) %RH. When the internal humidity of the culture apparatus is rapidly increased to 94%RH using a high-flow first preset flow rate of (150-200) ml/min, the second preset flow rate of (80-120) ml/min is adopted to transport gas into the culture apparatus, thereby maintaining the internal humidity of the culture apparatus within the range of (90-94) %RH. When the internal humidity of the culture apparatus is rapidly increased to 100%RH using a high-flow first preset flow rate of (200-300) ml/min, the second preset flow rate of (100-150) ml/min is adopted to transport gas into the culture apparatus, thereby maintaining the internal humidity of the culture apparatus within the range of (95-100) %RH.

In one embodiment, after the output end of the humidification module is connected to the gas inlet of the culture apparatus, the method further includes closing the hatch of the culture apparatus and verifying the sealing of the culture apparatus. After verifying the good sealing performance of the culture apparatus and the humidification module, a segmented flow rate control strategy is adopted to transport gas into the culture apparatus. Preferably, when the target humidity is 85 %RH, in the first stage, gas is transported into the culture apparatus at a flow rate of (100-150) ml/min. After passing through the humidification module, the gas rapidly exchanges with the internal gas of the culture apparatus through multiple gas outlets on the sealed metal groove, thereby quickly increasing the internal humidity of the culture apparatus to the target humidity of 85%RH or above. When detecting that the internal humidity of the culture apparatus has reached the target humidity, the gas transmission rate of the gas supply module is adjusted, and the second preset flow rate of (50-80) ml/min is adopted to transport gas into the culture apparatus, thereby maintaining the internal humidity of the culture apparatus within the target humidity range of (75-85) %RH. When the target humidity is 94%RH, in the first stage, gas is transported into the culture apparatus at a flow rate of (150-200) ml/min. After passing through the humidification module, the gas rapidly exchanges with the internal gas of the culture apparatus through multiple gas outlets on the sealed metal groove, thereby quickly increasing the internal humidity of the culture apparatus to the target humidity of 94%RH or above. When detecting that the internal humidity of the culture apparatus has reached the target humidity, the gas transmission rate of the gas supply module is adjusted, and the second preset flow rate of (80-120) ml/min is adopted to transport gas into the culture apparatus, thereby maintaining the internal humidity of the culture apparatus within the target humidity range of (90-94) %RH. When the target humidity is 95%RH, in the first stage, gas is transported into the culture apparatus at a flow rate of (200-300) ml/min. After passing through the humidification module, the gas rapidly exchanges with the internal gas of the culture apparatus through multiple gas outlets on the sealed metal groove, thereby quickly increasing the internal humidity of the culture apparatus to the target humidity of 95%RH or above. When detecting that the internal humidity of the culture apparatus has reached the target humidity, the gas transmission rate of the gas supply module is adjusted, and the second preset flow rate of (80-150) ml/min is adopted to transport gas into the culture apparatus, thereby maintaining the internal humidity of the culture apparatus within the target humidity range of (95-100) %RH. Based on the structural design of the sealed metal groove and gas outlets, the culture apparatus provided by the embodiment of the present disclosure can rapidly and uniformly increase the internal humidity of the culture apparatus, achieving a high saturation humidity of 90%RH or above.

In one embodiment, the flow control method for wet culture further includes: adjusting the operating state of the heating glass according to the cultivation mode of the culture apparatus and the gas supply module. When the cultivation mode of the culture apparatus is dry cultivation, regardless of whether the gas supply mode is self-mixed gas or pre-mixed gas, the heating glass is not controlled to heat. When the cultivation mode is wet cultivation and the gas supply mode is pre-mixed gas, the heating glass is controlled to heat to the same temperature range as the heating sheet.

As a preferred solution of the embodiment of the present disclosure, it also supports wet cultivation for two temperature-adjustable anti-condensation culture apparatuss simultaneously, to further increase the upper limit of cultivation capacity. To differentiate, the two culture apparatuss are named the main culture apparatus and the secondary culture apparatus. Specifically, by expanding the capacity of the humidification module, a humidification module can meet the environmental requirements for gas, humidity, and temperature for both culture apparatuss. Since the humidification module actually includes two sealing plugs, a humidification module can simultaneously meet the humidification needs of both culture apparatuss. The two culture apparatuss are arranged in parallel, and the humidification module is arranged between the two culture apparatuss. The two culture apparatuss and the humidification module are arranged in the same straight line. The connection between the humidification module and the two culture apparatuss is achieved through the sealing plugs, which provide wet cultivation for both culture apparatuss, thus enabling large-capacity wet cultivation at a lower cost.

In one embodiment, precise control of the gas flow ensures that the humidity within the culture apparatus remains in a saturated state, with humidity uniformity ≤ 5%RH.

As another preferred solution of the embodiment of the present disclosure, the embodiment can also realize simultaneous dry and wet cultivation. Specifically, the gas inlet at the connection between the secondary module and the humidification module is sealed off, at which point the secondary module pauses or terminates the wet cultivation and enters a dry cultivation state, and the main module continues wet cultivation according to the target humidity. Those skilled in the art will clearly understand that the flow control method for wet culture provided by the embodiment of the present disclosure is applicable to a temperature-adjustable anti-condensation culture apparatus, as described in embodiment 1. For convenience and simplicity, the specific working process of the above-described method applied to the culture apparatus can refer to the corresponding process in the aforementioned embodiment 1, and will not be repeated here.

In the embodiment of the present disclosure, a flow control device for wet culture is further provided, including a processor, a memory, and a computer program stored in the memory and configured to be executed by the processor, wherein the processor implements the flow control method for wet culture described above when executing the computer program.

In the embodiment of the present disclosure, a computer-readable storage medium is further provided, wherein the computer-readable storage medium includes a stored computer program, and when the computer program is run, the device in which the computer-readable storage medium is located is controlled to execute the flow control method for wet culture described above.

Exemplarily, the computer program can be divided into one or more modules, wherein one or more modules are stored in the memory and executed by the processor to complete the present disclosure. One or more modules can be a series of computer program instruction segments capable of completing specific functions, and the instruction segments are used to describe the execution process of the computer program in the flow control device for wet culture.

The flow control device for wet culture can be computing devices such as a desktop computer, a laptop, a handheld computer, and a cloud server. The flow control device for wet culture can include but is not limited to, a processor, a memory, and a display. A person skilled in the art can understand that the above components are merely exemplary of the flow control device for wet culture and do not constitute a limitation for the flow control device for wet culture. The flow control device for wet culture can include more or fewer components than the aforementioned components, or can combine certain components, or can include different components. For example, the flow control device for wet culture can further include input and output devices, network access devices, buses, etc.

The processor can be a central processing unit (CPU) or other general-purpose processors, digital signal processors (DSPs), application-specific integrated circuits (ASICs), field-programmable gate arrays (FPGAs), or other programmable logic devices, discrete gate or transistor logic devices, discrete hardware components, and the like. The general-purpose processor can be a microprocessor, or the processor can also be any conventional processor, and so on. The processor is the control center of the flow control device for wet culture and connects various parts of the flow control device for wet culture through various interfaces and circuits.

The memory can be configured to store the computer program and/or modules. The processor implements various functions of the flow control device for wet culture by running or executing computer programs and/or modules stored in the memory, and calling data stored in the memory. The memory can mainly include a program storage region and a data storage region. The program storage region can store an operating system and at least one application program required for functions (such as a sound playback function, a text conversion function, etc.). The data storage region can store data created according to the use of a mobile phone (such as audio data, text message data, etc.). Additionally, the memory can include high-speed random-access memory and non-volatile memory, such as hard disks, memory, plug-in hard disks, smart media cards (SMCs), secure digital (SD) cards, flash cards, at least one disk storage device, flash memory devices, or other volatile solid-state storage devices.

The integrated modules of the flow control device for wet culture can be stored on a computer-readable storage medium when implemented as software function units and sold or used as stand-alone products. Based on this understanding, all or part of the processes in the method embodiments of the present disclosure can also be implemented by instructing relevant hardware through a computer program. The computer program can be stored in a computer-readable storage medium, and when executed by a processor, the computer program can implement the steps of the various method embodiments described above. The computer program includes computer program code, and the computer program code can be in the form of source code, object code, executable files, or certain intermediate forms. The computer-readable medium can include: any entity or device capable of carrying the computer program code, recording medium, USB flash drive, mobile hard disk, magnetic disk, optical disk, computer memory, read-only memory (ROM), random access memory (RAM), electrical carrier signal, telecommunication signal and software distribution medium, etc. It should be noted that the content contained in the computer-readable medium can be appropriately increased or decreased according to the requirements of legislation and patent practice in different jurisdictions. For example, in some jurisdictions, according to legislation and patent practice, computer-readable media do not include electrical carrier signals and telecommunication signals. A person of ordinary skill in the art will be able to understand and implement them without inventive labor.

A flow control method for wet culture is provided in the embodiments of the present disclosure, where, under the wet culture method, gas is first transmitted into the culture apparatus at a relatively high gas flow rate. The humidified gas is delivered to the entire interior of the culture apparatus along the sealed metal groove, and the size of the gas outlets gradually increases in the direction of the hatch of the culture apparatus. Therefore, the gas after humidification treatment will not completely flow out at the flow distribution interface but can reach every corner inside the culture apparatus along the gas outlets, exchanging with the gas inside the culture apparatus. This ensures the uniformity of gas humidity and gas concentration inside the culture apparatus while rapidly achieving high saturation humidity. When the internal humidity of the culture apparatus reaches the target humidity, the gas flow rate is adjusted. A lower gas flow rate is used to transmit gas into the culture apparatus, ensuring that the internal humidity of the culture apparatus remains within the preset deviation range of the target humidity at all times.

The above is merely a preferred embodiment of the present disclosure. It should be noted that for those of ordinary skill in the art, several improvements and replacements can be made without departing from the technical principles of the present disclosure, and such improvements and replacements should also be regarded as within the protection scope of the present disclosure.

## Claims

1. A temperature-adjustable anti-condensation culture apparatus, comprising: an upper housing, a lower housing, a sealed metal groove, a culture turntable assembly, and a humidification module, wherein
a gas inlet is provided on an outer surface of a side edge of the lower housing and is connected to an external device;
the humidification module comprises a humidification water bottle, a humidification drawer configured for placing the humidification water bottle, and a humidification box sleeved over the humidification drawer, wherein a sealing plug is provided on the humidification box and is connected to the gas inlet;
a flow distribution interface is provided on an inner surface of a side edge of the lower housing at a position corresponding to the gas inlet and is connected to the sealed metal groove, wherein the sealed metal groove is arranged to surround an inner wall of the lower housing;
multiple gas outlets are provided on a surface of the sealed metal groove, starting from the flow distribution interface, wherein sizes of the gas outlets gradually increase in sequence in a direction toward a hatch of the culture apparatus;
the culture turntable assembly is located inside the lower housing and comprises, in sequence from top to bottom, a culture turntable for placing multiple culture dishes, a first heating sheet, and a temperature-equalizing plate, wherein the culture turntable, the first heating sheet, and the temperature-equalizing plate are attached to each other; an inner surface of a bottom of the lower housing is in closely attaching with the temperature-equalizing plate, wherein an outer surface of the bottom of the lower housing is provided with a second heating sheet; and
an upper surface of the lower housing is rigidly connected to the upper housing.

2. The temperature-adjustable anti-condensation culture apparatus according to claim 1, wherein a hatch structure comprising an L-shaped hatch and an L-shaped hatch cover panel is further provided at a front end of the culture apparatus, wherein
a front surface of the lower housing is rigidly connected to the L-shaped hatch, a longer-side panel of the L-shaped hatch is parallel to a bottom surface of the lower housing, and a shorter-side panel of the L-shaped hatch is directly attached with an outer surface of the lower housing;
the longer-side panel is provided with a perforation, wherein a size of the perforation is greater than a preset size range of the culture dishes; and
the L-shaped hatch cover panel is in closely attaching with the L-shaped hatch, wherein an inner surface of the L-shaped hatch cover panel is provided with a third heating sheet.

3. The temperature-adjustable anti-condensation culture apparatus according to claim 1, wherein the gas outlets of the sealed metal groove are symmetrically distributed on left and right sides relative to a centerline where the hatch of the culture apparatus is located.

4. The temperature-adjustable anti-condensation culture apparatus according to claim 1, wherein a diameter size range of the gas outlets is 0.2 mm to 3 mm.

5. The temperature-adjustable anti-condensation culture apparatus according to claim 1, wherein a snap is provided on a front surface of the humidification drawer, wherein
a water level observation window is provided below the snap, and
a semicircular opening is provided at a center of an upper portion on each of both sides of the humidification drawer.

6. The temperature-adjustable anti-condensation culture apparatus according to claim 1, wherein multiple fan-shaped culture dishes are provided on the culture turntable; and the fan-shaped culture dishes are closely arranged in a circular pattern around a center of the culture turntable, wherein each fan-shaped culture dish is provided with multiple culture microchambers for placing embryos, and the culture microchambers are arranged in a straight line.

7. A flow control method for wet culture, applicable to the temperature-adjustable anti-condensation culture apparatus according to any one of claims 1 to 6, comprising:
adding a target water amount to the humidification module according to a target humidity of the culture apparatus, wherein an input end of the humidification module is connected to a gas supply module, and an output end of the humidification module is connected to the gas inlet of the culture apparatus;
controlling the gas supply module to transmit gas to the humidification module at a first preset flow rate, so that the gas transmitted by the gas supply module is humidified in the humidification module and then reaches an interior of the culture apparatus via the gas outlets on the surface of the sealed metal groove, wherein the first preset flow rate has a flow rate range of 100 ml to 450 ml per minute; and
controlling, in response to detecting that an internal humidity of the culture apparatus has reached the target humidity, the gas supply module to transmit gas to the humidification module at a second preset flow rate, so that an internal humidity of the culture apparatus is maintained within a preset deviation range of the target humidity, wherein the second preset flow has a flow rate range of 50 ml to 230 ml per minute.

8. The flow control method for wet culture according to claim 7, wherein after the output end of the humidification module is connected to the gas inlet of the culture apparatus, the method further comprises: closing a hatch of the culture apparatus and verifying a sealing of the culture apparatus and the humidification module.

9. The flow control method for wet culture according to claim 7, wherein before controlling the gas supply module to transmit the gas to the humidification module at the first preset flow rate, the method further comprises real-time detection of an internal temperature of the culture apparatus and a temperature of a heater, wherein
the first heating sheet, a second heating sheet, a third heating sheet, and the heater are controlled to enter a heating mode until a preset temperature is reached; and
the first heating sheet, the second heating sheet, the third heating sheet, and the heater remain at the preset temperature when the wet culture apparatus operates.

10. A terminal device, comprising a processor, a memory, and a computer program stored in the memory and configured to be executed by the processor, wherein the processor implements the flow control method for wet culture according to any one of claims 7 to 9 when executing the computer program.
